# EUROPÄISCHE PATENTSCHRIFT

(11) **EP 1 013 298 B1**
(45) Veröffentlichungstag und Bekanntmachung des Hinweises auf die Patenterteilung: **20.04.2005**
(21) Anmeldenummer: 99125544.9
(22) Anmeldetag: 21.12.1999
(51) Int. Cl.: A61M 5/30, A61M 5/315, A61M 25/00

(54) **Ejektionsgerät zur Hochdruckejektion insbesondere einer medizinischen Flüssigkeit**
Ejection device for high pressure ejection of a liquid, in particular a medical liquid
Dispositif d'éjection pour éjection à haute pression d'un liquide en particulier médical

(30) Priorität: 21.12.1998 DE 19859133
(43) Veröffentlichungstag der Anmeldung: 28.06.2000
(73) Patentinhaber: Ferton Holding SA, 2800 Delémont (CH)
(72) Erfinder: Menne, Andreas, Dr., 88709 Meersburg (DE)
(74) Vertreter: Viering, Jentschura & Partner

(56) Entgegenhaltungen:
- WO-A-96/25190
- DE-A- 19 618 972
- US-A- 2 653 602
- US-A- 2 714 887
- US-A- 3 490 696
- US-A- 5 840 061

## Beschreibung

Die Erfindung betrifft ein Ejektionsgerät zur Hochdruckejektion einer Flüssigkeit oder einer Feststoffpartikel enthaltenden Flüssigkeit.

Aus der EP 0 771 219 sind Ejektionsgeräte bekannt, mit einem Gerätekopfteil und einer Druckkammer, die in eine distale Ejektionsöffnung ausmündet und die von einem Arbeitskolben begrenzt wird, von dem, durch Übertragung eines elastischen Stoßes auf das druckkammerferne Ende des Arbeitskolbens, eine Kompressionswelle übertragbar ist, durch welche das druckkammerseitige Ende des Arbeitskolbens in die Druckkammer unter Reduzierung von deren Volumen auslenkbar ist. Die Volumenreduktion der Druckkammer ist wesentlich kleiner als das Druckkammervolumen. Die Ejektionsgeräte haben ferner ein Geräteantriebsteil mit einem Antriebsstück, das in dem Geräteantriebsteil entlang einer Beschleunigungsstrecke bis zur Erzeugung des auf den Arbeitskolben übertragenen elastischen Stoßes angetrieben ist. Sie sind, wie aus der EP 0 771 219 ersichtlich ist, insbesondere zur Ejektion genau dosierter, kleinster Flüssigkeitsmengen, ggf. Kubikmillimeter, vorgesehen und eignen sich demnach insbesondere als medizinisches Geräte zur Injektion sehr kleiner und genau einzuhaltender Medikamenten-Flüssigkeitsdosen.

Das Ejizieren derart kleiner Flüssigkeitsmengen wird dadurch erzielt, daß von dem Antriebsstück lediglich ein elastischer Stoß auf den Arbeitskolben übertragen wird und dieser dann nicht weiter von dem Antriebsstück in die Druckkammer bewegt wird. Das heißt, nach erfolgter Stoßübertragung wird von dem Antriebsstück auf den Arbeitskolben keine äußere Antriebskraft mehr ausgeübt, so daß die von diesem begrenzte Flüssigkeit in der Druckkammer nicht wie bei einer gewöhnlichen Spritze ausgedrückt wird. Stattdessen wird das Ejizieren der Flüssigkeit lediglich von der durch die Stoßübertragung im Arbeitskolben angeregten Kompressionswelle erreicht, die sich auf die Flüssigkeit in der Druckkammer überträgt und eine Hochdruckejektion aus der Ejektionsöffnung bewirkt. Der Ejektionsdruck und damit das ejizierte Flüssigkeitsvolumen sind durch Einstellung der Antriebsgeschwindigkeit des Antriebsstücks genau einstellbar, da die Größe des übertragenen Impulses von dieser Antriebsgeschwindigkeit abhängt.

Gemäß den in der EP 0 771 219 beschriebenen Ejektionsgeräten ist das Druckkammervolumen wesentlich größer als das Hubvolumen des Arbeitskolbens, um unabhängig von der Größe der Druckkammer eine kleine, genau dosierte Menge der Flüssigkeit ejizieren zu können.

Um zu verhindern, daß das Antriebsstück nach der Stoßimpulsübertragung noch eine äußere Kraft auf den Arbeitskolben ausübt, kann das Antriebsstück beispielsweise nur bis zum Beginn der Stoßübertragung in Richtung des Arbeitskolbens angetrieben werden oder durch einen Anschlag an einer nach dem Stoß erfolgenden Kraftübertragung auf den Arbeitskolben gehindert sein.

Der Arbeitskolben ist vorzugsweise aus einem festen Material, von dem elastische Stoßwellen möglichst verlustfrei übertragen werden, wie z.B. einem Metallmaterial. Das Antriebsstück kann irgendein Bauteil sein, das entlang einer Beschleunigungsstrecke beschleunigbar ist, z.B. ein Antriebskolben, der in einem Antriebsrohr angetrieben beschleunigbar ist und bevorzugt zu dem Arbeitskolben koaxial ausgerichtet ist. Das Antriebsstück kann aber auch anders als ein Kolben gestaltet sein, beispielsweise als Platte oder Kipphebel, die/der auf den Arbeitskolben aufschlägt und dadurch den Antriebsstoß ausübt.

Das Antriebsstück kann pneumatisch, hydraulisch, mechanisch oder elektromagnetisch angetrieben sein. Hierbei kann der Antrieb des Antriebsstücks derart gestaltet sein, daß dieses lediglich für einen Antriebshub antreibbar ist. Bevorzugt ist jedoch das Antriebsstück zur sukzessiven Übertragung von Stoßimpulsen in periodischer Wiederholung antreibbar. Dadurch kann die Gesamtejektionsmenge in Abhängigkeit von der Anzahl der Wiederholungen gesteuert werden.

Bei der aus der EP 0 771 219 bekannten endoskopischen Variante ist der Arbeitskolben als sich in dem Endoskop-Katheter erstreckende Sonde mit einem proximalen Sondenkopf ausgebildet, der in einem Beschleunigungsrohr aufgenommen ist. In dem Beschleunigungsrohr ist ein Antriebskolben angeordnet, der auf den Sondenkopf auftrifft, um in der Sonde eine Kompressionswelle anzuregen, die ihrerseits ein Auslenken des im Katheter angeordneten Endes der Sonde in die von dem Katheterlumen gebildete Druckkammer bewirkt, so daß Flüssigkeit ejiziert wird.

Die aus der EP 0 771 219 bekannten Ejektionsgeräte sind aufgrund ihres integralen Aufbaus in ihren jeweiligen die zu ejizierende Flüssigkeit aufnehmenden Gerätekopfteilen schwierig zu reinigen und zu sterilisieren sowie steril zu halten.

Durch die Erfindung wird ein Ejektionsgerät geschaffen, dessen Gerätekopfteil in einfacher Weise gereinigt und sterilisiert werden kann und welches gleichzeitig einen ausreichenden Schutz gegen eine Kontamination der zu ejizierenden Flüssigkeit, insbesondere gegen eine durch den Geräteantriebsteil bedingte Kontamination, bietet.

Das erfindungsgemäße Ejektionsgerät hat die Merkmale im Anspruch 1

Durch die Ausbildung des Gerätekoptteils und des Geräteantriebsteils als lösbar miteinander verbundene, selbständige Geräteeinheiten, kann das Gerätekopfteil, von dem die zu ejizierende Flüssigkeit aufgenommen wird, in einfacher Weise separat gereinigt und sterilisiert werden. Das Geräteantriebsteil muß entweder nicht oder nur an seinem dem Gerätekopfteil zugewandten Stirnende sterilisiert werden. Dadurch daß die beiden Geräteeinheiten jeweils separat abgedichtet sind und daß insbesondere die Beschleunigungsstrecke des Geräteantriebsteils zu dem Gerätekopfteil hin abgedichtet ist, ist eine Kontamination der im Gerätekopfteil untergebrachten Flüssigkeit durch im Geräteantriebsteil vorliegende Schmutzpartikel oder Erreger ausreichend sicher unterbunden. Ferner ist es auch möglich, in einfacher und damit schneller Weise Kopfteile auszutauschen, in denen z.B. unterschiedliche Medikament-Flüssigkeiten aufgenommen sind.

Die Stoßübertragung erfolgt bei dem erfindungsgemäßen endoskopischen Ejektionsgerät über das Zwischenstück, auf welches das Antriebsstück aufschlägt. Durch das Aufschlagen des Antriebsstücks auf das Zwischenstück, wird in diesem zunächst eine elastische Stoßwelle angeregt, die sich in dem Zwischenstück fortpflanzt und von diesem ebenfalls als elastischer Stoß auf den Arbeitskolben übertragen wird, in dem seinerseits eine elastische Stoßwelle angeregt wird, die von dem Arbeitskolben auf die in der Druckkammer aufgenommene Flüssigkeit übertragen wird. Das Zwischenstück hat den Vorteil, daß es bei richtiger Wahl seiner Masse und bei bevorzugt spielfreier Anlage an dem Arbeitskolben zwar auf diesen den elastischen Stoß überträgt, selbst aber in einer Ruhestellung verharrt. Damit bleibt die über das Zwischenstück erreichte Abdichtung der Beschleunigungsstrecke auch bei der Stoßübertragung gewährleistet.

Dieses Dichtungsprinzip für einen Stoßantrieb ist auch in der DE 196 18 972 Al beschrieben, jedoch im Zusammenhang mit einem Nierensteinzertrümmerer.

Das Zwischenstück ist bevorzugt aus einem festen Material, welches elastische Stoßwellen möglichst verlustfrei übertragen kann, wie z.B. einem Metallmaterial. Das Zwischenstück ist ferner bevorzugt als kolbenförmiger Körper ausgebildet und in beide Axialrichtungen in seiner Bewegung durch einen Anschlag begrenzt, wobei zwischen dem arbeitskolbennahen Anschlag und dem Zwischenstück ein geringes Axialspiel vorgesehen ist, durch welches die Übertragung des Impulses von dem Zwischenstück auf den arbeitskolbennahen Anschlag verhindert wird und damit eine verlustfreie Übertragung des Stoßimpulses auf den Arbeitskolben gewährleistet ist.

Gemäß einer bevorzugten Ausführungsform weist das Gerätekopfteil eine Zwischenhülse auf, in welcher der Arbeitskolben aufgenommen ist und die einerseits über die lösbare Befestigungskupplung an dem Geräteantriebsteil befestigt ist und an der andererseits eine Kappe mit einer die Flüssigkeitsverbindung zur Ejektionsöffnung herstellenden Druckhohlraumaustrittsöffnung lösbar befestigt ist. Der Druckhohlraum kann in der Zwischenhülse lediglich ausgebildet sein, z.B. von einer Verlängerung der den Arbeitskolben aufnehmenden Ausnehmung in der Zwischenhülse. Er kann aber auch von einer wie vorstehend erläuterten Ausnehmung zusammen mit einer an diese angrenzende Ausnehmung in der Kappe ausgebildet sein.

Durch die zweiteilige Ausbildung des Gerätekopfteils kann dieses für Reinigungs- und Sterilisationszwecke in Einzelteile zerlegt werden und ist dann für Reinigungswerkzeuge besser zugänglich. Die Verbindung zwischen der Zwischenhülse und der Kappe kann z.B. durch eine Steckverbindung, einen Bajonettverschluß oder eine Schraubverbindung erreicht werden.

Obwohl auch durch einen engen Trennspalt zwischen der Kappe und der Zwischenhülse eine Fortpflanzung der Druckwelle und damit ein Austreten von Flüssigkeit aus der Druckkammer im ausreichenden Maße verhindert werden kann, ist vorteilhafterweise eine die Trennfuge zwischen Kappe und Zwischenhülse abdichtende elastische Dichtung vorgesehen, von welcher eine Kontamination der Flüssigkeit, z.B. durch Krankheitserreger, noch besser verhindert wird. Ferner ist bevorzugt auch eine die Trennfuge zwischen der Zwischenhülse und dem Geräteantriebsteil abdichtende elastische Dichtung vorgesehen, so daß auch zwischen diesen beiden Teilen ein Eindringen von Erregern wirksam unterbunden ist.

Für einen periodischen Betrieb des erfindungsgemäßen Ejektionsgeräts ist ein Flüssigkeits-Nachfüllanschluß vorgesehen, der vorteilhafterweise an der Zwischenhülse ausgebildet ist und der über den Trennspalt zwischen dem Arbeitskolben und der Zwischenhülse mit dem Druckhohlraum verbunden ist. Der Trennspalt zwischen dem Arbeitskolben und der Zwischenhülse genügt zum einen zur Herstellung einer ausreichenden Flüssigkeitsverbindung zwischen der Druckkammer und dem Flüssigkeits-Nachfüllanschluß und ist zum anderen ausreichend klein, so daß sich die in der Druckkammer angeregte Druckwelle nur in geringem Maße durch den Spalt hindurch nach hinten zu dem Flüssigkeits-Nachfüllanschluß fortpflanzen kann. Ferner kann damit auch ein separater Zuführkanal entfallen, der zusätzlich zu sterilisieren und gegebenenfalls mit einem Rückschlagventil zu versehen wäre. Der Flüssigkeits-Nachfüllanschluß ist z.B. in Form eines Stutzens ausgebildet, der seitlich in die Zwischenhülse geschraubt oder gesteckt ist. Hierzu weist die Zwischenhülse bevorzugt einen in ihrer Längsmitte ausgebildeten radialen Ringbund auf, der einen Sockel für den Flüssigkeits-Nachfüllanschluß bildet und der ferner eine gute Handhabe zur Befestigung der Zwischenhülse inkl. der Kappe an dem Antriebsteil darstellt. Der Flüssigkeits-Nachfüllanschluß kann jedoch auch an der Kappe vorgesehen sein, in welcher dann ein ggf. von einem Rückschlagventil beherrschter Kanal ausgebildet ist, über den die Druckkammer mit dem Nachfüllanschluß in Verbindung steht.

Zur Verbesserung der Effektivität des Ejektionsgeräts ist der Arbeitskolben von der Druckkammer aus betrachtet nach dem Flüssigkeits-Nachfüllanschluß mit einem elastischen Dichtungselement umfangsseitig gegen die Zwischenhülse abgedichtet. Durch diese Dichtung wird ein Flüssigkeitsaustritt in Richtung des Geräteantriebsteils unterbunden, so daß letztlich nur durch die Ejektionsöffnung aus der Druckkammer Flüssigkeit austreten kann. Zusätzlich wird durch dieses Dichtungselement eine Fortpflanzung der Druckwelle in dem Trennspalt zwischen dem Arbeitskolben und der Zwischenhülse unterbunden, wodurch sekundär ein Ejizieren der Flüssigkeit aus der Druckkammer nach hinten verhindert wird.

Obwohl das elastische Dichtungselement z.B. in einer inneren Ringnut der Zwischenhülse gegen den Arbeitskolbenumfang abdichtend angeordnet sein kann, ist bevorzugt an dem druckkammerfernen Ende des Arbeitskolbens ein radialer Ringbund ausgebildet, zwischen dem und einer hülsenseitigen Schulter das elastische Dichtungselement auf der der Druckkammer zugewandten Seite des Ringbunds angeordnet ist. Dies hat den Vorteil, daß der Anpreßdruck des elastischen Dichtungselements im Moment des Ejizierens erhöht wird, da der Antriebskolben mit dem Ringbund durch den auf den Kolben übertragenen Stoß in Richtung zu der hülsenseitigen Schulter gestoßen wird. Damit wird eine noch bessere Abdichtung der Druckkammer erzielt. Das Dichtungselement hat den weiteren Vorteil, daß der Arbeitskolben immer in seine vor dem Stoß vorliegende axiale Ausgangsposition zurückgeführt wird, welche, wie oben erläutert, bevorzugt eine an dem Zwischenstück anliegende Position ist. Hierdurch wird auch das Zwischenstück gegen den arbeitskolbenfernen Anschlag gedrückt, so daß das für den einwandfreien Betrieb erforderliche Axialspiel zwischen dem Zwischenstück und dem arbeitskolbennahen Anschlag gewährleistet ist. Um einen Flüssigkeitsaustritt auch im Ruhezustand sicherzustellen, ist das Dichtungselement vorteilhafterweise unter Vorspannung zwischen dem Ringbund und der hülsenseitigen Schulter angeordnet. Die Wirkung des Zurückführens des Arbeitskolbens zusammen mit dem Zwischenstück in die jeweilige Ausgangsposition kann auch durch das wie oben erwähnte, in der hülsenseitigen Ringnut angeordnete Dichtungselement erreicht werden, wenn dieses unter Vorspannung in die Nut eingesetzt ist, so daß sich der Arbeitskolben in Axialrichtung relativ zu dem Dichtungselement nicht verschieben kann.

Rings des Trennspalts zwischen dem Arbeitskolben und dem Zwischenstück ist bevorzugt ein Ausgleichshohlraum vorgesehen, welcher mit einem Gas, wie z.B. Luft, gefüllt ist. Im Falle daß der Arbeitskolben von dem Zwischenstück bei der Stoßübertragung abhebt, entsteht durch den sich dazwischen ergebenden Hohlraum ein Unterdruck, der die Bewegung des Arbeitskolbens beeinträchtigen kann. Durch den Ausgleichshohlraum, der größer ist als der zwischen dem Zwischenstück und dem Kolben entstehende Hohlraum, fällt diese Druckreduzierung jedoch so gering aus, daß die Bewegung des Arbeitskolbens nicht beeinträchtigt wird.

Nach einer Ausführungsform ist das erfindungsgemäße Ejektionsgerät als endoskopisches Ejektionsgerät ausgebildet. Hierzu ist an das Gerätekopfteil ein Endoskop-Katheter angeschlossen, der ein die Flüssigkeit führendes Katheterlumen und die Ejektionsöffnung aufweist, wobei die Druckkammer von dem Katheterlumen und dem Druckhohlraum ausgebildet wird, der dann zwischen dem Katheterlumen und dem Arbeitskolben angeordnet ist.

Bei dieser Ausführungsform hat die Ausbildung zumindest eines Teils der Druckkammer in dem Kopfteil den Vorteil, daß durch die Bewegung des Arbeitskolbens eventuell hervorgerufene Reibverschleißrückstände hauptsächlich im Kopfteil und nicht im Katheterlumen auftreten, so daß zum einen die Gefahr eines Ejizierens von Verschleißpartikeln reduziert wird und daß zum anderen die Verschleißrückstände einfacher zu entfernen sind, da das Kopfteil im Gegensatz zu dem einzuführenden, langgestreckten, engen Katheter für Reinigungsgeräte wesentlich besser zugänglich gestaltbar ist. Insgesamt ist es möglich, alle beweglichen Teile, wie z.B. noch ein Rückschlagventil, von dem ein Zurücksaugen von Luft in die Druckkammer verhindert wird, in dem Kopfteil vorzusehen, so daß der Endoskop-Katheter lediglich eine Flüssigkeitsführungsfunktion hat und demnach mit besonders kleinen Abmessungen ausgebildet werden kann. Es können zudem auch problemlos unterschiedliche Katheter an das Kopfteil angeschlossenen werden, welche einen beliebigen Innendurchmesser aufweisen, ohne daß die Funktion des endoskopischen Geräts beeinträchtigt wird.

Der Katheter ist z.B. über eine Schraub- oder Steckverbindung an dem Gerätekopfteil, bevorzugt an deren dem Arbeitskolben gegenüberliegenden Ende, befestigt. Bei der mehrteiligen Ausbildung des Gerätekopfteils mit der Kappe ist der Katheter demnach vorteilhafterweise an der Kappe angebracht. Der Endoskopkatheter kann als starrer oder als flexibler Katheter ausgebildet sein. Ferner kann der Endoskop-Katheter derart ausgebildet sein, daß er in den Arbeitskanal eines Endoskops einführbar ist. Hinsichtlich eines endoskopischen Einsatzes sind das Ejektionsgerät und das Endoskop auch bevorzugt zu einer Geräteeinheit zusammengefaßt.

Im Hinblick auf die bevorzugte Verwendung des erfindungsgemäßen Geräts kommen als Materialien der die Druckkammer umgebenden Begrenzungswände und damit der die Druckkammer begrenzenden Teile, wie der Zwischenhülse, der Kappe, dem Arbeitskolben und der Dichtungen, insbesondere leicht zu sterilisierende und leicht steril zu haltende, d.h. z.B. besonders glatte, Materialien in Frage, wie, abgesehen von den Dichtungen, z.B. polierter Stahl oder poliertes Titan oder Legierungen daraus. Die Materialien sollten zudem auch inert gegenüber der zu ejizierenden Flüssigkeit sein.

Zu Reinigungs- und Sterilisationszwecken kann die Befestigungskupplung gelöst und das Gerätekopfteil von dem Antriebsteil abgenommen werden. Der Arbeitskolben kann dann zusammen mit dem ihn umgebenden Dichtungselement aus der Zwischenhülse entnommen und das Dichtungselement von dem Arbeitskolben abgestreift werden. Desweiteren können der die Kappe und der daran befestigte Katheter sowie die Zwischenhülse und die Kappe voneinander gelöst werden.

Um zu Verhindern, daß das Antriebsstück seine Ausgangsposition ungewollt verläßt, ist eine Haltevorrichtung vorgesehen, mittels deren das Antriebsstück in der Ausgangsposition gehalten wird. Die Haltevorrichtung kann z.B. eine Klemmvorrichtung sein, mittels deren das Antriebsstück durch Klemmkräfte gehalten ist, welche von den Antriebskräften überwindbar sind. Vorteilhafterweise ist zum Halten des Antriebsstücks eine Magnethalterung vorgesehen, welche z.B. von einem Elektromagneten, bevorzugt aber von einem Dauermagneten gebildet ist. An dem Antriebsstück kann als Gegenstück zu der Magnethalterung ein magnetisches Teil, wie ein magnetisierbares Metallteil angebracht sein. Bevorzugt ist jedoch das gesamte Antriebsstück aus einem magnetisierbaren Werkstoff, wie z.B. Stahl.

Die Erfindung wird nachfolgend anhand bevorzugter Ausführungsformen mit Bezugnahme auf die Zeichnung erläutert. In der Zeichnung zeigen:
Figur 1 einen Längsschnitt eines Ejektionsgeräts gemäß einer Ausführungsform der Erfindung und
Figur 2 eine vergrößerte Darstellung des vorderen Teils des in Figur 1 dargestellten Ejektionsgeräts.

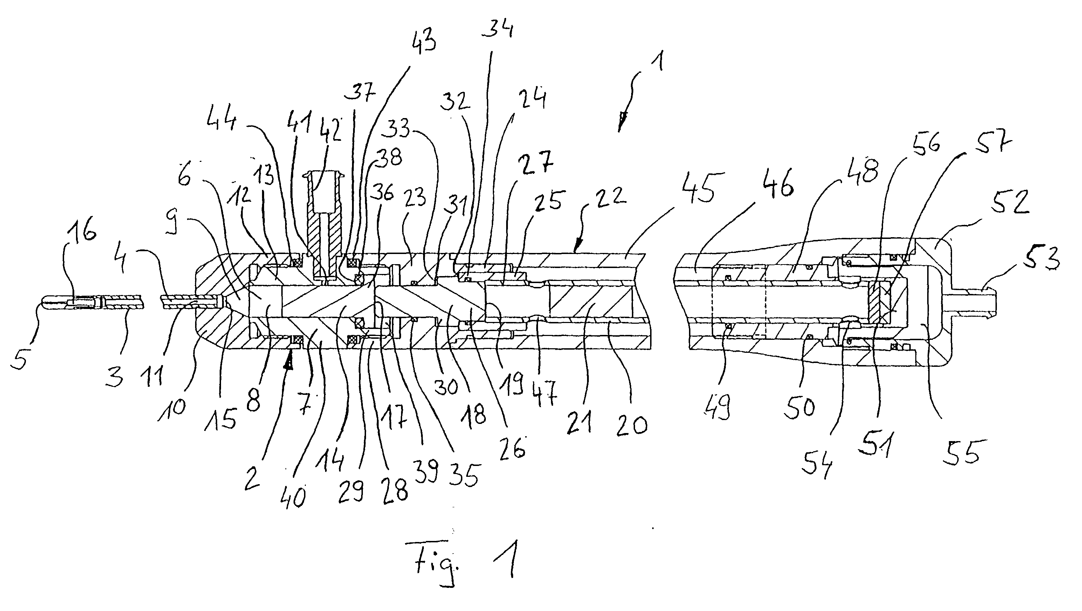

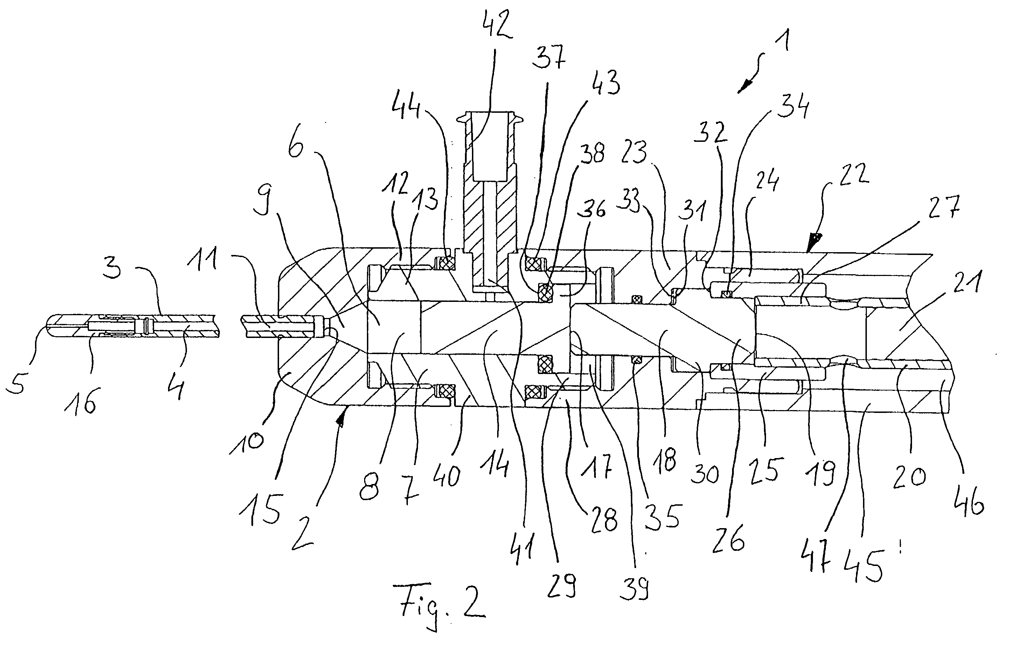

Figuren 1 und 2 zeigen in jeweils einem Längsschnitt ein erfindungsgemäßes Ejektionsgerät 1 zur Hochdruckejektion einer Flüssigkeit in einer endoskopischen Variante. Das Ejektionsgerät 1 weist ein Gerätekopfteil 2 auf, an das ein Endoskop-Katheter 3 in Form eines biegsamen Rohrs oder Schlauchs angeschlossen ist, der ein die Flüssigkeit führendes Katheterlumen 4 in Form eines Kanals und eine Ejektionsöffnung 5 aufweist. In dem Gerätekopfteil 2 ist ein Druckhohlraum 6 ausgebildet, der abschnittsweise von einem von einer Zwischenhülse 7 ausgebildeten kreiszylindrischen Hohlraum 8 und einer an diesen bündig angrenzenden Ausnehmung 9 in einer auf die Zwischenhülse 7 axial aufgeschraubten Kappe 10 gebildet wird. Die Zwischenhülse 7 und die Kappe 10 sind beide Teile des Gerätekopfteils 2. Der Druckhohlraum 6 und das Katheterlumen 4 bilden eine die Flüssigkeit aufnehmende Druckkammer 11.

Die Kappe 10 ist an ihrem der Zwischenhülse 7 zugewandten Endabschnitt 12 mit einem axial verlaufenden Innengewinde versehen, das mit einem axialen Außengewinde auf dem der Kappe 10 zugewandten Endabschnitt 13 der Zwischenhülse 7 zusammenwirkt. Der Druckhohlraum 6 und damit die Druckkammer 11 werden hülsenseitig von einem in dem Hohlraum 8 der Zwischenhülse 7 angeordneten kreiszylindrischen Arbeitskolben 14 begrenzt. Der Druckhohlraum 6 mündet kappenseitig in eine Druckhohlraumaustrittsöffnung 15. Hierzu ist die Ausnehmung 9 in der Kappe 10 in Axialrichtung wegweisend von dem Druckhohlraum 6 kegelförmig verjüngt. An die Druckhohlraumaustrittsöffnung 15 ist der Endoskop-Katheter 3 angeschlossen, dessen Katheterlumen 4 über die Druckhohlraumaustrittsöffnung 15 mit dem Druckhohlraum 6 in Verbindung steht. Der Katheter 3 ist hierzu stirnseitig in die Kappe 10 eingesteckt oder eingeschraubt. Am Ejektionsende des Endoskop-Katheters 3 ist ferner eine Ejektionsdüse 16 ausgebildet.

An dem vom Druckhohlraum 6 abgewandten Stirnende 17 des Arbeitskolbens 14 liegt ein Zwischenstück 18 stirnseitig an. Das Zwischenstück 18 ist als im wesentlichen kreiszylindrischer Zwischenkolben ausgebildet und zu dem Arbeitskolben 14 koaxial angeordnet. Das Zwischenstück 18 ist an seiner von dem Arbeitskolben 14 abgewandten axialen Seite mit einem stirnseitig an dem Stirnende 19 des Zwischenstücks 18 anliegenden kreiszylindrischen Antriebsrohr 20 verbunden, welches zu dem Zwischenstück 18 und damit zu dem Arbeitskolben 14 koaxial angeordnet ist. In dem Antriebsrohr 20 ist ein Antriebsstück in Form eines kreiszylindrischen Antriebskolbens 21 in Längsrichtung des Antriebsrohrs 19 frei bewegbar angeordnet. Der Antriebskolben 21 ist, wie später noch genauer erläutert, in Richtung zu dem Arbeitskolben 14 beschleunigbar, um über das dazwischenangeordnete Zwischenstück 18 auf den Arbeitskolben 14 einen elastischen Stoß zu übertragen, der eine Auslenkung des druckkammerseitigen Endes des Arbeitskolbens 14 in die Druckkammer 11 bewirkt.

Der Antriebskolben 21, das Antriebsrohr 20 und das Zwischenstück 18 sind Teile eines Geräteantriebsteils 22, an dem das Gerätekopfteil 2, wie nachfolgend erläutert, lösbar befestigt ist.

Das Geräteantriebsteil 22 weist eine zu der Zwischenhülse 7 koaxial angeordnete Aufnahmemuffe 23 auf, in der das Zwischenstück 18 angeordnet ist. In den von der Zwischenhülse 7 abgewandten Endabschnitt 24 der Aufnahmemuffe 23 ist eine Stopfbuchse 25 eingesteckt, in welcher der von dem Arbeitskolben 14 abgewandte Endabschnitt 26 des Zwischenstücks 18 und der dem Arbeitskolben 14 zugewandte Endabschnitt 27 des Antriebsrohrs 20 aufgenommen ist. Die Stopfbuchse 25 ist dicht gegen die Aufnahmemuffe 23 gepreßt; alternativ kann sie gegen letztere mittels einer Dichtung abgedichtet sein. Die Aufnahmemuffe 23 ist an ihrem der Zwischenhülse 7 zugewandten Endabschnitt 28 mit einem Innengewinde versehen, und die Zwischenhülse 7 ist an ihrem der Aufnahmemuffe 23 zugewandten Endabschnitt 29 mit einem Außengewinde versehen und in die Aufnahmemuffe 23 geschraubt. Bei dieser Ausführungsform wird die lösbare Verbindung zwischen dem Geräteantriebsteil 22 und dem Gerätekopfstück 2 somit von der Schraubverbindung zwischen der Zwischenhülse 7 und der Aufnahmemuffe 23 gebildet.

Das Zwischenstück 18 weist einen radialen Ringbund 30 auf, der mit geringem Axialspiel und radial spielfrei in einer Ausnehmung 31 aufgenommen ist, deren axiale Begrenzungsflächen von einem inneren Randabschnitt des dem Zwischenstück 18 zugewandten Stirnendes 32 der Stopfbuchse 25 und einer von der Aufnahmemuffe 23 ausgebildeten inneren Schulter 33 gebildet sind und deren radiale Begrenzungsflächen von einem zylindrischen Begrenzungswandabschnitt der Aufnahmemuffe 23 gebildet ist. Das Zwischenstück 18 ist in Schlagrichtung des Antriebskolbens 21 gesehen jeweils vor und hinter seinem Ringbund 30 mit einem O-Ring 34, 35 gegen die Stopfbuchse 25 bzw. gegen die Aufnahmemuffe 23 abgedichtet.

Der Arbeitskolben 14 weist an seinem dem Zwischenstück 18 zugewandten Ende einen radialen Ringbund 36 auf, zwischen welchem und einer hülsenseitigen, inneren Schulter 37 auf der von dem Zwischenstück 18 abgewandten Seite ein O-Ring 38 angeordnet ist. Von diesem O-Ring 38 wird der Arbeitskolben 14 einerseits gegen die Zwischenhülse 7 abgedichtet und andererseits axial gegen das Zwischenstück 18 vorgespannt, so daß dieses mit seinem Ringbund 30 stirnseitig gegen das ihm zugewandte Stirnende 32 der Stopfbuchse 25 gedrückt wird. Zwischen der dem Zwischenstück 18 zugewandten Stirnfläche des Ringbunds 36 an dem Arbeitskolben 14 und einer diesem gegenüberliegenden inneren Stirnfläche der Aufnahmemuffe 23 ist rings des Trennspalts zwischen dem Arbeitskolben 14 und dem Zwischenstück 18 eine dieses umgebende, gasgefüllte Ringkammer 39 ausgebildet, welche einen Ausgleichshohlraum darstellt.

Die Zwischenhülse 7 weist einen nach außen weisenden Ringbund 40 auf, der zwischen den beiden Außengewinden ausgebildet ist. In dem Ringbund 40 ist ein radial verlaufendes Durchgangsloch 41 ausgebildet, in das ein Stutzen 42 geschraubt ist, der einen Flüssigkeits-Nachfüllanschluß bildet. Das Durchgangsloch 41 steht über den zwischen dem Arbeitskolben 14 und der Zwischenhülse 7 vorliegenden Spalt mit dem Druckhohlraum 6 und damit mit der Druckkammer 11 in Verbindung. Über den Stutzen 42 kann somit der Druckkammer 11 Flüssigkeit zugeführt werden. Vor und hinter dem Ringbund 40 der Zwischenhülse 3 ist an dieser jeweils ein O-Ring 43, 44 gegen den Ringbund 40 anliegend angeordnet. Mittels des vor dem Ringbund 40 angeordneten O-Rings 43 ist die Zwischenhülse 7 umfangsseitig gegen die Aufnahmemuffe 23 abgedichtet, und mittels des nach dem Ringbund 40 angeordneten O-Rings 44 ist die Zwischenhülse 7 umfangsseitig gegen die Kappe 10 abgedichtet.

Auf den von der Zwischenhülse 7 abgewandten Endabschnitt 24 der Aufnahmemuffe 23 ist ein das Antriebsrohr 20 aufnehmendes Gehäuse in Form eines kreiszylindrischen Aufnahmerohrs 45 aufgesteckt, wobei das Aufnahmerohr 45 dicht gegen die Aufnahmemuffe 23 gepreßt ist. Zwischen dem Aufnahmerohr 45 und dem Antriebsrohr 20 ist eine Ringkammer 46 ausgebildet, die über benachbart zu der Stopfbuchse 25 in dem Antriebsrohr 20 seitlich ausgebildete Öffnungen 47 mit dem Inneren des Antriebsrohrs 20 in Verbindung steht.

Die Ringkammer 46 wird an ihrem von dem Gerätekopfteil 2 abgewandten Ende axial von einer Dichthülse 48 begrenzt, die das Antriebsrohr 20 umgebend axial in das Aufnahmerohr 45 geschraubt ist und mittels O-Ringen 49, 50 gegen das Antriebsrohr 20 und das Aufnahmerohr 45 abgedichtet ist.

Das von dem Gerätekopfteil 2 abgewandte Ende des Antriebsrohrs 20 ist mit einem Verschlußdeckel 51 dicht verschlossen. Das von dem Gerätekopfteil 22 abgewandte Ende des Aufnahmerohrs 45 ist mit einem Stopfen 52 dicht verschlossen, an dem ein Anschlußstutzen 53 für die Zuführung von Preßluft ausgebildet ist. Das Antriebsrohr 20 ist benachbart zu dem Verschlußdeckel 51 mit seitlichen Öffnungen 54 versehen, die über eine zwischen dem Stopfen 52, der Dichthülse 48, dem Aufnahmerohr 45 und dem Antriebsrohr 20 ausgebildete Kammer 55 mit der Öffnung des Stutzens 53 in Verbindung stehen. In dem Verschlußdeckel 51 sind eine starre Platte 56 und eine elastische Platte 57 angeordnet, die zusammen als axialer, gepufferter Anschlag für den Antriebskolben 21 dienen. Hierzu sind die beiden Platten 56, 57 unmittelbar hintereinander angeordnet, wobei die starre Platte 56 zu dem Antriebskolben 21 näher liegt. Die elastische Platte 57 ist aus einem elastischen Kunststoff oder aus Gummi und dient als Puffer. Die starre Platte 56 ist aus einem dauermagnetischen Material, d.h. die Platte 56 bildet einen Dauermagneten. Der Antriebskolben 21 ist aus einem magnetisierbaren Material, wie z.B. Stahl. Damit bildet die starre Platte 56 für den Antriebskolben 21 eine Magnethalterung, an der der Antriebskolben 21 in seiner Ausgangsposition gegen ungewolltes Bewegen gesichert gehalten ist. Die magnetischen Kräfte zwischen der starren Platte 56 und dem Antriebskolben 21 sind so bemessen, daß sie von den Antriebskräften überwunden werden.

Im folgenden wird die Funktionsweise des vorstehend beschriebenen endoskopischen Ejektionsgeräts 1 beschrieben. Mittels einer nicht dargestellte Pumpe wird dem Ejektionsgerät 1 über den Anschlußstutzen 53 Druckluft zugeführt, die über die Kammer 55 und die Öffnungen 54 in das Innere des Antriebsrohrs 20 geleitet wird, wo sie auf den Antriebskolben 21 trifft, welcher hierdurch in Richtung zu dem Zwischenstück 18 beschleunigt wird. Die von dem Antriebskolben 21 in dem Antriebsrohr 20 verdrängte Luft wird durch die Öffnungen 47 in die Ringkammer 46 gepreßt. Der Antriebskolben 21 wird nur bis zum Anschlagen an das Zwischenstück 18 beschleunigt, d.h. von einer durch die zugeführte Preßluft bewirkten Kraft angetrieben. Die in der Ringkammer 46 komprimierte Luft strömt dann durch die Öffnungen 47 zurück in das Antriebsrohr 20 und drückt den Kolben 21 zurück in seine Ausgangsstellung.

Mit dem Anschlagen des Antriebskolbens 21 auf das Zwischenstück 18 wird in diesem eine Kompressionswelle angeregt, die das Zwischenstück 18 durchläuft und von diesem auf den Arbeitskolben 14 als elastischer Stoß übertragen wird, der sich ebenfalls als elastische Kompressionswelle in dem Arbeitskolben fortpflanzt. Durch das Axialspiel zwischen dem Ringbund 30 des Zwischenstücks 18 und der inneren Schulter 33 der Aufnahmemuffe 23 wird verhindert, daß der Stoß von dem Zwischenstück 18 auf die Aufnahmemuffe 23 übertragen wird. Das Axialspiel wird durch den hinter dem Ringbund 36 des Arbeitskolbens 14 angeordneten O-Ring 38 gewährleistet, von dem der Arbeitskolben 14 gegen das Zwischenstück 18 gedrückt wird, welches seinerseits mit seinem Ringbund 30 unter Aufrechterhaltung des für den Betrieb notwendigen Axialspiels stirnseitig gegen die Stopfbuchse 25 gedrückt wird.

Die wie oben erläutert auf den Arbeitskolben 14 übertragene Kompressionswelle durchläuft denselben und bewirkt eine Auslenkung des dem Druckhohlraum 6 und damit der Druckkammer 11 zugewandten Endes des Arbeitskolben 14 in die Druckkammer 11 hinein. Hierdurch erhöht sich der Druck in der Druckkammer 11, wodurch die darin aufgenommene Flüssigkeit über die Ejektionsöffnung 5 ausgespritzt wird.

In dem Kopfteil 2 kann ein Rückschlagventil angeordnet sein, von dem ein Ansaugen von Luft über die Ejektionsöffnung 5 in das Katheterlumen 4 verhindert wird. Das Rückschlagventil kann z.B. als ein die Druckhohlraumaustrittsöffnung 15 beherrschendes Ventil in der Kappe 10 vorgesehen sein. Ferner kann auch in dem Stutzen 42 ein Rückschlagventil vorgesehen sein, welches lediglich ein Zuführen von Flüssigkeit aus einem Vorratsbehälter in die Druckkammer 11 zuläßt.

Die Anordnung des O-Rings 38 zwischen dem Ringbund 36 und der inneren Schulter 37 hat den weiteren Vorteil, daß der O-Ring 38 bei der Stoßübertragung von dem Zwischenstück 18 auf den Arbeitskolben 14 zusammengepreßt wird, so daß der Arbeitskolben 14 zum Zeitpunkt des Druckanstiegs in der Druckkammer 11 besser gegen die Zwischenhülse 3 abgedichtet ist.

Die das Zwischenstück 18 umgebende Ringkammer 39 sorgt im Falle daß der Arbeitskolben 14 von dem Zwischenstück 18 abhebt für einen Druckausgleich, so daß kein solcher Unterdruck entsteht, der andernfalls die Verlagerung des Arbeitskolbens 14 und damit den einwandfreien Betrieb des Ejekionsgeräts 1 verhindern könnte.

Die zweiteilige Ausbildung des erfindungsgemäßen endoskopischen Ejektionsgeräts 1 mit einem Gerätekopf- 2 und einem Geräteantriebsteil 22 hat den Vorteil, daß das Gerätekopfteil 2, in dem die Unterbringung und Zuführung der Flüssigkeit vorgesehen sind, separat von dem Geräteantriebsteil 22 vorbereitet und sterilisiert werden kann. Ferner muß bei einem Defekt eines der beiden Teile 2, 22 nicht das komplette Gerät 1, sondern lediglich das defekte Teil ausgetauscht werden.

Durch die Ausbildung des Geräteantriebsteils 22 und des Gerätekopfteils 2 als separate und gekapselte, d.h. abgedichtete, Geräteeinheiten wird zudem ein verbesserter Schutz gegen die Kontamination der im Gerätekopfteil 2 untergebrachten Flüssigkeit erreicht.

## Patentansprüche

1. Ejektionsgerät (1) zur Hochdruckejektion einer Flüssigkeit oder einer Partikel enthaltenden Flüssigkeit, mit
einem Gerätekopfteil (2), welches aufweist:
zumindest einen Teil einer Druckkammer (11), die in eine distale Ejektionsöffnung (5) ausmündet, und
einen Arbeitskolben (14), welcher die Druckkammer an deren proximalem Ende begrenzt, und
einem Geräteantriebsteil (22), welches aufweist:
ein Antriebsstück (21), das in dem Geräteantriebsteil (22) entlang einer Beschleunigungsstrecke (20) bis zur Erzeugung eines auf den Arbeitskolben (14) übertragenen elastischen Stoßes angetrieben ist, und
ein am distalen Ende der Beschleunigungsstrecke (20) angeordnetes, vom Arbeitskolben (14) und vom Antriebsstück (21) separates Zwischenstück (18), von welchem die Beschleunigungsstrecke an ihrem distalen Ende dicht verschlossen wird und auf welches das Antriebsstück (21) unter Erzeugung des elastischen Stoßes aufschlägt **dadurch gekennzeichnet, daß** sowohl das Gerätekopfteil als auch das Geräteantriebsteil jeweils als selbständige Geräteeinheiten ausgebildet sind, wobei
das Gerätekopfteil (2) und das Geräteantriebsteil (22) separat abgedichtet sind und über eine lösbare Befestigungskupplung derart aneinander befestigt sind, daß von dem Zwischenstück (18) unter Erzeugung einer Kompressionswelle im Arbeitskolben, durch welche das druckkammerseitige Ende des Arbeitskolbens (14) in die Druckkammer (11) unter einer Reduzierung von deren Volumen, wobei die Volumenreduktion der Druckkammer wesentlich kleiner ist als das Druckkammervolumen, auslenkbar ist, der elastische Stoß auf das druckkammerseitige Ende des Arbeitskolbens (14) übertragbar ist.

2. Ejektionsgerät (1) nach Anspruch 1, **dadurch gekennzeichnet, daß** das Gerätekopfteil (2) eine Zwischenhülse (7) aufweist, in der der Arbeitskolben (14) aufgenommen ist und die einerseits über die lösbare Befestigungskupplung an dem Geräteantriebsteil (22) befestigt ist und an der andererseits eine Kappe (10) mit einer die Flüssigkeitsverbindung zur Ejektionsöffnung (5) herstellenden Druckhohlraumaustrittsöffnung (15) lösbar befestigt ist.

3. Ejektionsgerät (1) nach Anspruch 2, **dadurch gekennzeichnet, daß** die Zwischenhülse (7) gegen die Kappe (10) und gegen das Geräteantriebsteil (22) jeweils mit einer elastischen Dichtung (44, 43) abgedichtet ist.

4. Ejektionsgerät (1) nach Anspruch 2 oder 3, **dadurch gekennzeichnet, daß** an der Zwischenhülse (7) ein Flüssigkeits-Nachfüllanschluß (42) ausgebildet ist, der über den Spalt zwischen dem Arbeitskolben (14) und der Zwischenhülse (7) mit dem Druckhohlraum (6) verbunden ist.

5. Ejektionsgerät (1) nach einem der Ansprüche 2 bis 4, **dadurch gekennzeichnet, daß** der Arbeitskolben (14) mit einem elastischen Dichtungselement (38) gegen die Zwischenhülse (7) abgedichtet ist.

6. Ejektionsgerät (1) nach Anspruch 5, **dadurch gekennzeichnet, daß** der Arbeitskolben (14) an seinem druckkammerfernen Ende einen radialen Ringbund (36) aufweist, zwischen dem und einer hülsenseitigen Schulter (37) das elastische Dichtungselement (38) angeordnet ist.

7. Ejektionsgerät (1) nach einem der Ansprüche 1 bis 6, **dadurch gekennzeichnet, daß** rings des Trennspalts zwischen dem Arbeitskolben (14) und dem Zwischenstück (18) ein Ausgleichshohlraum (39) vorgesehen ist.

8. Ejektionsgerät (1) nach einem der Ansprüche 1 bis 7, **dadurch gekennzeichnet, daß** an das Gerätekopfteil (2) ein Endoskop-Katheter (3) angeschlossen ist, der ein die Flüssigkeit führendes Katheterlumen (4) und die Ejektionsöffnung (5) aufweist, und daß die Druckkammer (11) von dem Katheterlumen (4) und dem Druckhohlraum (6) gebildet wird.

9. Ejektionsgerät (1) nach einem der Ansprüche 1 bis 8, **gekennzeichnet durch** ein Endoskop, mit dem das Ejektionsgerät (1) unter Ausbildung einer Geräteeinheit verbunden ist.

10. Ejektionsgerät (1) nach einem der Ansprüche 1 bis 9, **dadurch gekennzeichnet, daß** das Antriebsstück (21) in seiner Ausgangsposition von einer Magnethalterung gehalten ist.

## Claims

1. An ejection device (1) for high-pressure ejection of a liquid or a liquid containing solid particles, comprising:
a head unit (2) including:
at least a part of a pressure chamber (11) which opens into a distal ejection opening (5) and
a working piston (14) which delimits the pressure chamber at its proximal end,
and a drive unit (22) including:
a drive member (21) which is driven along an acceleration portion (20) within the drive unit (22) until generation of an elastic impact to be transmitted to the working piston (14), and
an intermediary member (18) arranged at the distal end of the acceleration portion (20), being separate from the working piston (14) and the drive member (21), by means of which the acceleration portion is tightly sealed at the distal end thereof and on which the drive member (21) abuts, thereby generating the elastic impact,
**characterized in that** both the head unit (2) and the drive unit (22) are formed as independent units wherein the head unit (2) and the drive unit (22) are separately sealed and mounted to each other by a separable mounting coupling such that said intermediary member (18) is capable of transmitting the elastic impact to the pressure chamber-facing end of the working piston (14), thereby generating a compression wave in the working piston, by means of which the the pressure chamber-facing end of the drive piston (14) is displaceable into the pressure chamber (11) so that the volume thereof is reduced, wherein the reduction in volume of the pressure chamber (11) is significantly smaller than the volume of the pressure chamber.

2. The ejection device (1) according to claim 1, **characterized in that** the head unit (2) comprises an intermediary sleeve (7) which accommodates the working piston (14) and which, on one hand, is mounted to the drive unit (22) by means of the separable mounting coupling and on which, on the other hand, is a cap (10) removably mounted, said cap (10) comprising a pressure cavity outlet (15) that provides a fluid connection with the ejection opening (5).

3. The ejection device (1) according to claim 2, **characterized in that** the intermediary sleeve (7) is sealed against the cap (10) and the drive unit (22) by means of an elastic seal (44 and 43), respectively.

4. The ejection device (1) according to claim 2 or 3, **characterized in that** the intermediary sleeve (7) is formed with a liquid filling connection (42) which communicates with the pressure cavity (6) via the gap formed between the working piston (14) and the intermediary sleeve (7).

5. The ejection device (1) according to any one of claims 2 to 4, **characterized in that** the working piston (14) is sealed against the intermediary sleeve (7) by means of an elastic sealing element (38).

6. The ejection device (1) according to claim 5, **characterized in that** the working piston (14) comprises a radial flange (36) at its end facing away from the pressure chamber, with the elastic sealing element (38) being arranged between said flange and a shoulder (37) formed at the intermediary sleeve (7).

7. The ejection device (1) according to any one of claims 1 to 6, **characterized in that** a compensation cavity (39) is provided around the gap between the working piston (14) and the intermediary member (18).

8. The ejection device (1) according to any one of claims 1 to 7, **characterized in that** the head unit (2) is connected to an endoscopic catheter (3) which comprises a lumen (4) enabling the passage of liquid therethrough as well as the ejection opening (5), and that the pressure chamber (11) is formed by the lumen (4) of the catheter and by the pressure cavity (6).

9. The ejection device (1) according to any one of claims 1 to 8, **characterized by** an endoscope to which the ejection device (1) is connected to form a single unit.

10. The ejection device (1) according to any one of claims 1 to 9, **characterized in that** the drive member (21) is held in its starting position by a magnetic fixing device.

## Revendications

1. Dispositif d'éjection (1) pour l'éjection à haute pression d'un liquide ou d'un liquide contenant des particules, comportant
une partie de tête (2) du dispositif, qui comporte:
au moins une partie d'une chambre de pression (11), qui débouche dans une ouverture distale d'éjection (5), et
un piston de travail (14), qui limite la chambre de pression au niveau de son extrémité proximale, et
une partie d'entraînement (22) du dispositif, qui comporte:
un organe d'entraînement (21), qui est entraîné dans la partie d'entraînement (22) du dispositif le long d'une section d'accélération (20) jusqu'à la production d'un choc élastique transmis au piston de travail (14), et
un organe intercalaire (18), qui est disposé sur l'extrémité distale de la section d'accélération (20), est séparé du piston de travail (14) et de l'organe d'entraînement (21) et ferme d'une manière étanche la section d'accélération au niveau de son extrémité distale et que l'organe d'entraînement (21) frappe en produisant le choc élastique,
**caractérisé en ce qu'**aussi bien la partie de tête du dispositif que la partie d'entraînement du dispositif sont agencées respectivement sous la forme d'unités autonomes du dispositif,
la partie de tête (2) du dispositif et la partie d'entraînement (22) du dispositif étant étanchéifiées séparément et pouvant être fixées l'une à l'autre par l'intermédiaire d'un accouplement de fixation amovible de telle sorte que le choc élastique peut être transmis à l'extrémité, située du côté de la chambre de pression, du piston de travail (14) par l'organe intercalaire (18) moyennant la production dans le piston de travail, d'une onde de compression, grâce à laquelle l'extrémité, située du côté de la chambre de pression, du piston de travail (14) peut être déviée dans la chambre de pression. (11) moyennant une réduction du volume de cette dernière, la réduction de volume de la chambre de pression étant nettement inférieure au volume de la chambre de pression.

2. Dispositif d'éjection (1) selon la revendication 1, **caractérisé en ce que** la partie de tête (2) du dispositif comporte une douille intermédiaire (7), dans lequel est logé le piston de travail (14) et qui d'une part est fixée sur la partie d'entraînement (22) du dispositif par l'intermédiaire de l'accouplement de fixation amovible et sur laquelle d'autre part est fixé de façon amovible un capuchon (10) comportant une ouverture (15) de sortie de la cavité sous pression, qui établit une liaison fluidique avec l'ouverture d'éjection (5).

3. Dispositif d'éjection (1) selon la revendication 2, **caractérisé en ce que** la douille intercalaire (7) est appliquée de façon étanche contre le capuchon (10) et contre la partie d'entraînement (22) du dispositif, respectivement au moyen de garnitures d'étanchéité élastiques respectives (44, 43).

4. Dispositif d'éjection (1) selon la revendication 2 ou 3, **caractérisé en ce que** sur la douille intercalaire (7) est formé un raccord (42) de remplissage complémentaire en liquide, qui est relié à la cavité sous pression (6) par l'intermédiaire de la fente présente entre le piston de travail (14) et la douille intercalaire (7).

5. Dispositif d'éjection (1) selon l'une des revendications 2 à 4, **caractérisé en ce que** le piston de travail (14) est fermé de façon étanche par un élément d'étanchéité élastique (38) vis-à-vis de la douille intercalaire (7).

6. Dispositif d'éjection (1) selon la revendication 5, **caractérisé en ce que** le piston de travail (14) comporte, sur son extrémité éloignée de la chambre de pression, un collet annulaire radial (36), l'élément d'étanchéité élastique (38) étant disposé entre ce collet annulaire et un épaulement (37) situé sur la douille.

7. Dispositif d'éjection (1) selon l'une des revendications 1 à 6, **caractérisé en ce qu'**une cavité de compensation (39) est prévue autour de la fente de séparation entre le piston de travail (14) et l'organe intercalaire (18).

8. Dispositif d'éjection (1) selon l'une des revendications 1 à 7, **caractérisé en ce qu'**à la partie de tête (2) du dispositif est raccordé un cathéter d'endoscope (3), qui comporte une lumière de cathéter (4), qui guide le liquide, et l'ouverture d'éjection (5), et que la chambre de pression (11) est formée par la lumière (4) du cathéter et par la cavité sous pression (6).

9. Dispositif d'éjection (1) selon l'une des revendications 1 à 8, **caractérisé par** un endoscope, auquel le dispositif d'éjection (1) est relié en formant une unité de dispositif.

10. Dispositif d'éjection (1) selon l'une des revendications 1 à 9, **caractérisé en ce que** l'organe d'entraînement (21) est retenu dans sa position de départ par un dispositif de retenue magnétique.
